# EUROPÄISCHE PATENTANMELDUNG

(11) **EP 2 111 857 A1**
(43) Veröffentlichungstag der Anmeldung: **28.10.2009**
(21) Anmeldenummer: 08155167.3
(22) Anmeldetag: 25.04.2008
(51) Int. Cl.: A61K 9/70, A61K 31/4468

(54) **Transdermales therapeutisches System zur Verabreichung von Fentanyl oder einem Analogstoff hiervon**

(71) Anmelder: Acino AG, 83714 Miesbach (DE)
(72) Erfinder: Salman, Nouha, 81373, München (DE); Teutsch, Ingo, 81371, München (DE)
(74) Vertreter: Best, Michael

(57) **Zusammenfassung**

Offenbart wird ein transdermales therapeutisches System zur Verabreichung eines Wirkstoffes durch die Haut umfassend:
(a) eine Rückschicht
(b) ein auf der Rückschicht befindliches Reservoir, umfassend den Wirkstoff, Polyisobutylen, einen Gelbildner und einen Weichmacher, wobei ein Teil des Wirkstoffs in dem Reservoir in Form ungelöster Partikel vorliegt und der Gehalt an Gelbildner in dem Reservoir höchstens 4 Gew.-% beträgt (bezogen auf das Gesamtgewicht des Reservoirs),

wobei der Wirkstoff Fentanyl oder ein Analogstoff davon ist.

## Beschreibung

Gegenstand der vorliegenden Anmeldung ist ein System zum transdermalen Verabreichen von Fentanyl oder einem Analogstoff hiervon für therapeutische Zwecke.

Fentanyl und dessen Analogstoffe, insbesondere Alfentanil, Carfentanil, Lofentanil, Remifentanil, Sufentanil, Trefentanil und ähnliche Verbindungen sind wirksame synthetische Opiate. Fentanyl und dessen Analogstoffe sind hochwirksam und werden schnell metabolisiert. Problematisch bei Fentanyl ist, dass es einen relativ engen therapeutischen Index besitzt. Bei Überschreiten der Grenzwerte treten unerwünschte Nebeneffekte auf, insbesondere eine Beeinträchtigung der Atmung, die - wenn nicht geeignete Gegenmaßnahmen ergriffen werden - zum Tod führen kann. Die Wirkstoffe sind relativ teuer und es besteht ein sehr hohes Missbrauchsrisiko. Deshalb müssen Fentanylpflaster einerseits eine sehr präzise gesteuerte Freisetzung des Wirkstoffes gewährleisten und andererseits soll das Produkt so geschaffen sein, dass der Wirkstoff hieraus nicht leicht zu Missbrauchszwecken entfernt werden kann.

Ein Transdermalpflaster ist typischerweise eine kleine klebende Bandage, welche den anzugebenden Wirkstoff enthält. Diese Bandagen können verschiedene Formen und Größen haben. Der einfachste Typ ist ein Klebe-Monolith, welcher einen Wirkstoffvorrat (Reservoir) auf einem Träger umfasst. Das Reservoir wird typischerweise aus dem Wirkstoff in einem pharmazeutisch akzeptablen druckempfindlichen Klebstoff gebildet. Es kann aber auch aus einem nicht klebenden Material geformt sein, dessen Haut-Kontaktfläche mit einer dünnen Schicht eines geeigneten Klebstoffes versehen ist. Die Geschwindigkeit, mit welcher der Wirkstoff dem Patienten von diesen Pflastern verabreicht wird, kann bezüglich der Durchlässigkeit der Haut für den Wirkstoff von Person zu Person sowie von Hautstelle zu Hautstelle in bestimmten Grenzen variieren.

Komplexere Pflaster sind Mehrfachlaminate oder Pflaster mit Wirkstoffvorrat (der gegebenenfalls in einer Flüssigkeit gelöst vorliegen kann), in welchen zwischen dem Reservoir und dem die Haut kontaktierenden Klebstoff eine die Wirkstoff-Freisetzung steuernde Membran angeordnet sein kann. Diese Membran dient dazu, die Auswirkungen von Variationen der Hautdurchlässigkeit durch Herabsetzung der in vitro- und in vivo-Abgaberate des Wirkstoffes aus dem Pflaster zu kontrollieren und gegebenenfalls zu vermindern.

Das Reservoir der Transdermalpflaster kann den Wirkstoff entweder vollständig gelöst im Vorrat enthalten oder er kann einen Überschuss ungelösten Wirkstoffes über dessen Sättigungskonzentration hinaus enthalten (Depot-Pflaster). Die Anwesenheit von ungelöstem Wirkstoff oder anderen Bestandteilen in einem Pflaster kann bei der Lagerung sowie beim Gebrauch allerdings Stabilitäts- und andere Probleme aufwerfen. Eine Schwierigkeit besteht auch darin, dass sichergestellt werden muss, dass sich ausreichend Wirkstoff aus dem festen Depot nachlöst, um den abgegebenen Wirkstoff zu ersetzen. Wirkstoffpflaster, deren Reservoir feste Wirkstoffteilchen aufweist, werden im Stand der Technik daher häufig als nachteilig angesehen.

Aus dem Stand der Technik sind viele verschiedene transdermale Pflaster für die Verabreichung von Fentanyl bekannt. Die WO 02/074286 beschreibt ein transdermales Pflaster mit einem Reservoir enthaltend Fentanyl, wobei das Reservoir eine polymere Zusammensetzung, bevorzugt Polyacrylat, in einheitlichem Phasenzustand aufweist, die frei ist von nicht gelöstem Wirkstoff. Eine Übersättigung soll hier ausdrücklich vermieden werden.

Es gibt viele Versuche, Fentanylpflaster auch auf der Basis einer Matrixschicht aus Polyisobutylen herzustellen. Erste derartige Versuche werden bereits in dem Grundpatent zu Fentanylpflastern, dem US-A 4,588,580, beschrieben. Diese Druckschrift offenbart ein transdermales therapeutisches System mit einer Polyisobutylenmatrix und Mineralöl, die eine 2%ige Beladung von Fentanyl als ungelöstem Feststoff enthält. Das System zeigte allerdings eine zeitabhängige Freisetzung, das heißt man hat die ersten 20 Stunden nach der Verabreichung einen Anstieg der Konzentration des Fentanyls im Blut, und anschließend einen langsamen Abfall der Konzentration. Dies ist ein ungünstiges Freisetzungsprofil, und in der Folgezeit ging die Entwicklung daher von Polyisobutylenmatrices weg bzw. falls Polyisobutylenmatrices verwendet wurden, versuchte man, den Wirkstoff vollständig in der Polyisobutylenmatrix zu lösen.

Ein transdermales therapeutisches System mit einer Polyisobutylenmatrix ist in Roy et al., Journal of Pharmaceutical Sciences, Vol. 85, Nr. 5, Mai 1996, Seiten 491 bis 495 beschrieben. Es wird gezeigt, dass bei Konzentrationen von Fentanyl in der Polyisobutylenmatrix von mehr als 4% Wirkstoff ausfällt, und dies wurde offensichtlich von Roy et al. als negativ angesehen. Roy et al. schlägt für Pflaster mit geringer Wirkstoffbeladung an Fentanyl Polysilikonpflaster vor und keine Polyisobutylenpflaster.

In der EP 1 625 854, der US 2007/0009588 und der US 2006/0013865 werden dementsprechend Polyisobutylenmatrices vorgeschlagen, bei denen sorgfältig darauf zu achten ist, dass der Wirkstoff in der Polyisobutylenmatrix vollständig gelöst vorliegt. Das Auftreten von Kristallen in der Matrix wird als negativ angesehen. Die dort offenbarten Systeme zeigen allerdings das gleiche Problem wie die feststoffhaltigen Systeme der US 4,588,560, nämlich eine zeitabhängige Freisetzungsgeschwindigkeit, bei der die Konzentration des Fentanyls im Blut eines Patienten innerhalb der ersten 20 Stunden nach der Verabreichung zunimmt, dann aber nicht wie gewünscht über einen Zeitraum von mindestens 2, besser mindestens 3 Tagen, konstant bleibt, sondern über diesen Zeitraum abfällt.

Die DE 198 37 902 offenbart transdermale therapeutische Systeme auf der Basis von Polyisobutylen, die insbesondere zur Verabreichung von Clonidin geeignet sind, unter den dort genannten Wirkstoffen findet sich aber auch Fentanyl. Beispiele für Fentanylpflaster finden sich in dieser Druckschrift nicht, ein Hinweis darauf, dass bei den dort offenbarten Pflastern der Wirkstoff als Feststoff vorhanden sein soll, findet sich in der Druckschrift ebenfalls nicht. Die Druckschrift enthält keine in vivo-Untersuchungen zur Freisetzung des Wirkstoffs aus den Pflastern. Die Polyisobutylenschicht dieser Pflaster enthält mindestens 5 Gew.-% eines Füllstoffs.

Trotz all dieser Versuche gibt es bisher noch kein Pflaster auf Basis von Polyisobutylen, das die Erfordernisse der Zulassungsbehörden hinsichtlich der Wirkstofffreisetzung bzw. der erzielbaren Blutplasmaspiegel erfüllt und daher vermarktbar wäre.

Im Hinblick auf den Stand der Technik stellt sich die Aufgabe, ein transdermales therapeutisches System zur Verabreichung von Fentanyl oder eines Analogstoffs davon durch die Haut zur Verfügung zu stellen, das auf der Basis von Polyisobutylen aufgebaut ist und das die Probleme des Standes der Technik nicht zeigt. Das Pflaster soll insbesondere in der Lage sein, Fentanyl bzw. einen Analogstoff davon möglichst schnell und insbesondere gleichmäßig über einen langen Zeitraum freizusetzen und die zeitabhängige Freisetzungsgeschwindigkeit, die sich bei den Polyisobutylensystemen des Standes der Technik zeigt, nicht aufweisen. Der Blutplasmaspiegel sollte über einen möglichst langen Zeitraum, bevorzugt aber über einen Zeitraum von etwa 30 Stunden nach der Verabreichung bis etwa 70 Stunden nach der Verabreichung, möglichst konstant bleiben.

Gegenstand der vorliegenden Erfindung ist daher ein transdermales therapeutisches System zur Verabreichung eines Wirkstoffes durch die Haut umfassend:
(a) eine Rückschicht
(b) ein auf der Rückschicht befindliches Reservoir, umfassend den Wirkstoff, Polyisobutylen, einen Gelbildner und einen Weichmacher, wobei ein Teil des Wirkstoffs in dem Reservoir in Form ungelöster Partikel vorliegt und der Gehalt an Gelbildner in dem Reservoir höchstens 4 Gew.-% beträgt (bezogen auf das Gesamtgewicht des Reservoirs),
wobei der Wirkstoff Fentanyl oder ein Analogstoff davon ist.

Neben Fentanyl sind erfindungsgemäß bevorzugt Analogstoffe des Fentanyls, wie Alfentanil, Lofentanil, Remifentanil oder Sufentanil, wobei es sich bei dem Wirkstoff besonders bevorzugt um Fentanyl oder Sufentanil handelt. Die Erfindung wird im Folgenden im Wesentlichen anhand des Fentanyls erläutert. Die Ausführungen gelten aber entsprechend auch für die Analogstoffe des Fentanyls.

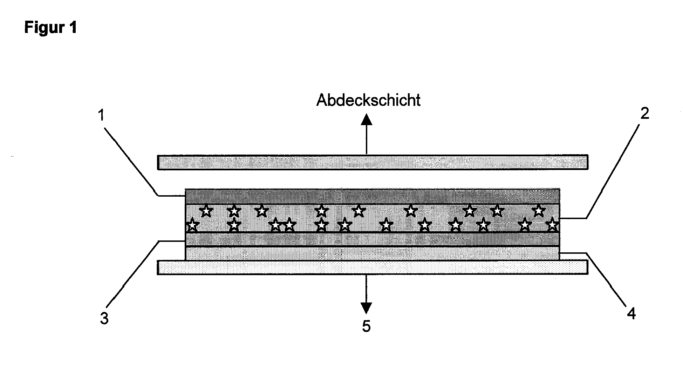

Der Aufbau eines bevorzugten transdermalen therapeutischen Systems im Querschnitt ist in Figur 1 dargestellt. Abgedeckt wird das transdermale therapeutische System durch eine Abdeckschicht. Auf dem bei der Anwendung der Haut gegenüber befindlichen Ende des Pflasters befindet sich die Rückschicht (1). An der der menschlichen Haut beim Gebrauch zugewandten Seite der Rückschicht (1) befindet sich das Reservoir (2). In einer bevorzugten Ausführungsform kann das transdermale therapeutische System eine Membran (3) aufweisen. Die Membran (3) ist zwischen dem Reservoir (2) und der Haut, auf die das Pflaster aufgebracht werden soll, angeordnet. Falls eine Membran (3) vorhanden ist, befindet sich zwischen der Membran (3) und der Haut bevorzugt eine Klebstoffschicht (4), die wirkstofffrei ist, alternativ aber nicht bevorzugt kann in diesem Fall eine Klebstoffschicht auch neben der Membran angebracht sein und diese z.B. kreisförmig umschließen.

Auf der dem Reservoir (2) gegenüberliegenden Seite der Klebstoffschicht (4) befindet sich noch eine Abziehschicht (5), die vor Gebrauch des transdermalen therapeutischen Systems abgezogen wird.

In einer bevorzugten Ausführungsform ist bei dem transdermalen therapeutischen System bei der der Rückschicht entgegengesetzten Seite des Reservoirs (2) eine Membran (3) vorgesehen, die die Freisetzung des Wirkstoffes kontrolliert. Hauptzweck der Membran ist es, die in vivo- und in vitro-Freisetzungsrate des Wirkstoffes aus dem Pflaster zu reduzieren. Dadurch können Unterschiede in der Permeabilität für den Wirkstoff durch die Haut ausgeglichen werden. Bevorzugt handelt es sich bei der Membran um eine mikroporöse Membran.

Geeignete Membranen sind im Stand der Technik bekannt. In einer bevorzugten Ausführungsform kann die Membran Polypropylen oder Polyethylenvinylacetat enthalten oder daraus bestehen. Ein besonders bevorzugtes Material für die Membran ist eine mikroporöse Polypropylenfolie.

Die Dicke der Membran ist nicht besonders eingeschränkt und kann z.B. im Bereich von 10 µm bis 100 µm, bevorzugt kleiner 50 µm, z.B. bei etwa 25 µm, liegen. Die Porengröße liegt bevorzugt im Bereich von 0,001 bis 0,025 µm², z.B. im Bereich von 0,002 bis 0,011 µm², insbesondere bei etwa 0,005 µm². Die Form der Poren ist ebenfalls nicht besonders eingeschränkt, bevorzugt ist eine rechteckige Form.

Ein typisches Beispiel für eine geeignete Membran ist daher eine mikroporöse Polypropylenfolie mit einer Dicke von etwa 25 µm und einer Porengröße von etwa 0,12 µm x 0,04 µm, wie sie unter dem Handelsnamen Celgard 2400 der Firma Celgard LLC, Charlotte, USA vertrieben wird.

Gegebenenfalls kann die Membran nach bekannten Verfahren vorbehandelt werden.

Zwischen der Membran und der Abzugsfolie befindet sich bevorzugt eine wirkstofffreie Klebstoffschicht (4), die die Haftung des Pflasters auf der Haut ermöglicht. Alternativ kann die Klebstoffschicht auch um die Membran herum angeordnet sein. Diese Klebstoffschicht kann aus verschiedenen, an sich bekannten Haftklebstoffen bestehen, wobei bevorzugt Polyisobutylen, aber auch andere Stoffe wie Polybuten, verschieden Harze aus Mineralölprodukten oder geeignete Polyacrylate verwendet werden können. Falls für die Klebstoffschicht ein Polyisobutylen als Haftkleber verwendet wird, wird bevorzugt das gleiche Polyisobutylen verwendet, das auch für das Reservoir eingesetzt wird. Die Klebstoffschicht kann übliche Zusatzstoffe für Klebstoffschichten bei Wirkstoffpflastern enthalten. Die Dicke der Klebstoffschicht (Trockendicke) kann in einem Bereich von etwa 10 µm bis etwa 300 µm, vorzugsweise zwischen etwa 70 µm und etwa 140 µm variieren. Wenn man die Menge der Klebstoffschicht auf Gewicht pro Fläche des Pflasters bezieht, beträgt die Menge der Klebstoffschicht bevorzugt etwa 10 mg/10 cm² bis etwa 50 mg/10 cm², vorzugsweise etwa 20 bis etwa 40 mg/10 cm² (anwendungsfertiges, das heißt trockenes, Pflaster).

Eine solche zusätzliche Klebstoffschicht kann selbstverständlich auch bei der Ausführungsform des erfindungsgemäßen Pflasters ohne Membran vorhanden sein. Falls bei dieser Ausführungsform das Reservoir bereits eine ausreichende Klebrigkeit aufweist, kann auf die zusätzliche Klebstoffschicht auch verzichtet werden.

Auf der Klebstoffschicht bzw. dem selbstklebenden Reservoir ohne Membran befindet sich eine Abziehschicht (release liner), die in Figur 1 mit der Nummer 5 gekennzeichnet wurde. Diese Abziehschicht ist bevorzugt aus polymerem Material hergestellt, das gegebenenfalls auch metallisiert sein kann. Beispiele für bevorzugt eingesetzte polymere Materialien sind Polyurethane, Polyvinylacetat, Polyvinylidenchlorid, Polypropylen, Polycarbonat, Polystyrol, Polyethylen, Polyethylenterephthalat, Polybutylenterephthalat sowie gegebenenfalls mit entsprechenden Polymeren oberflächenbeschichtetes Papier. Bevorzugt handelt es sich hierbei um eine einseitig oder beidseitig fluorierte oder silikonisierte Abziehschicht. Besonders bevorzugt sind handelsübliche fluorierte oder silikonisierte Polyesterfolien, wie die einseitig silikonisierten Handelsprodukte Primeliner 100 µm und Perlosic LF 75 µm (Firma Loparex, NL und Perlen Converting AG, Schweiz).

Das Reservoir (3) umfasst den Wirkstoff, bevorzugt Fentanyl, Polyisobutylen, einen Gelbildner und einen Weichmacher, wobei die einzelnen Komponenten so aufeinander abgestimmt sind, dass ein Teil des Wirkstoffs (Fentanyl) in dem Reservoir in Form ungelöster Partikel vorliegt, der Gehalt an Fentanyl also oberhalb der Sättigungslöslichkeit des Fentanyls in dem Reservoir liegt, so dass nicht alles Fentanyl in Lösung geht. Dies gilt bei Raumtemperatur, also bei etwa 25°C, bevorzugt aber sowohl bei Raumtemperatur als auch bei der Anwendungstemperatur (Hauttemperatur), also bei etwa 34°C (die Hauttemperatur liegt etwas unterhalb der Körpertemperatur).

Bei dem Weichmacher handelt es sich um eine prinzipiell bekannte Verbindung, die im Stand der Technik in transdermalen therapeutischen Systemen als Weichmacher eingesetzt wird. Der Weichmacher dient bevorzugt auch noch dazu, die Penetration des Wirkstoffs durch die Haut zu verbessern, und in einer besonders bevorzugten Ausführungsform reguliert er die Löslichkeit des Wirkstoffs in dem Reservoir, so dass ein bestimmter Gehalt an Wirkstoff in Lösung gehalten wird.

Bei dem Weichmacher handelt es sich bevorzugt um Mineralöl, Leinsamenöl, Octylpalmitat, Squalen, Squalan, Silikonöl, Isobutylmyristat, Isostearylalkohol und Oleylalkohol, wobei Mineralöle als Weichmacher bevorzugt sind. Diese auch als dünnflüssige Paraffine bezeichneten Öle sind farblose, klare Kohlenwasserstoffe. Sie werden aus den oberhalb von etwa 300°C siedenden Destillationsfraktionen des Erdöls gewonnen und durch Abkühlen von festen Kohlenwasserstoffen befreit. Durch Extrahieren mit Lösungsmittel sowie durch Behandlung mit Bleicherden und/oder Schwefelsäure werden sie raffiniert. Geeignete Mineralöle sind sowohl in chemischer wie auch in biologischer Hinsicht stabil und verhindern bakterielles Wachstum. Durch geeignete Fraktionierung können Mineralöle gewonnen werden, die etwa bei Körpertemperatur, also bei etwa 35 bis 37°C flüssig sind, bei tieferen Temperaturen, insbesondere bei Temperaturen unter 20°C fest sind. Die Auswahl eines Mineralöls mit einem Verflüssigungspunkt von etwa 30-35°C ist bevorzugt.

Der Weichmacher ist in dem Reservoir bevorzugt in einer Menge im Bereich von 10 bis 60 Gew.-%, stärker bevorzugt von 25 bis 50 Gew.-%, insbesondere im Bereich von 30 bis 40 Gew.-%, vorhanden (bezogen auf das Gesamtgewicht des Reservoirs).

In dem transdermalen therapeutischen System muss das Reservoir eine Menge an Fentanyl oder einem Analogstoff davon enthalten, die ausreichend ist um Schmerzfreiheit bei einem Menschen zu induzieren und für mindestens zwei Tage, bevorzugt mindestens drei Tage, aufrechtzuerhalten (bezogen auf den Zeitpunkt der Verabreichung des Pflasters). Bevorzugt enthält das Reservoir eine Menge an Fentanyl oder Analogstoff davon, die ausreichend ist, um Schmerzfreiheit zu induzieren und für einen Zeitraum von mindestens drei Tagen, insbesondere von drei bis sieben Tagen aufrechtzuerhalten.

Die absolute Menge an einzusetzendem Wirkstoff hängt von verschiedenen Faktoren, insbesondere von der Größe des einzusetzenden Pflasters ab. Bevorzugt enthält das transdermale therapeutische System den Wirkstoff in einer Menge von 5 bis 20 Gew.-%, stärker bevorzugt in einer Menge von 5 bis 15 Gew.-%, bezogen auf das Gesamtgewicht des Reservoirs. Es ergeben sich bevorzugt Flächengewichte im Bereich von 20 bis 100 g/m², stärker bevorzugt 25 bis 80 g/m², insbesondere im Bereich von 30 bis 70 g/m². Das Reservoir weist bevorzugt eine Dicke (Trockendicke) im Bereich von 20 bis 400 µm, stärker bevorzugt im Bereich von 30 bis 200 µm, insbesondere im Bereich von 40 bis 100 µm auf.

Das Reservoir des erfindungsgemäßen Pflasters beinhaltet auch einen Gelbildner. Hierbei handelt es sich bevorzugt um einen Gelbildner mit partikulärer Struktur, der auf seiner Oberfläche eine hohe Konzentration polarer Gruppen aufweist. Diese verursachen zu den Ölen hin entsprechend hohe Grenzflächenspannungen, die durch Agglomeration der Partikel untereinander zu Gelgerüsten teilweise kompensiert werden. Demzufolge sind die Gelgerüste immer um so fester, je größer der Polaritätsunterschied zwischen den Ölen und der Gerüstbildneroberfläche ist. Bevorzugt wird erfindungsgemäß als Gelbildner hochdisperses Siliziumdioxid oder kolloidales Siliziumdioxid eingesetzt. Die Größe der Partikel bewegt sich bevorzugt im Nanobereich und liegt z.B. im Bereich von 400 bis 1500 nm, insbesondere im Bereich von 500 bis 1000 nm. Kolloidales Siliziumdioxid wird beispielsweise unter der Bezeichnung Cab-O-Sil^{®} vertrieben und ist ein bekanntes Verdickungsmittel für Mineralöl. Ein anderes Beispiel für einen geeigneten Gelbildner ist Bentonit. Auch das als Gelbildner bekannte Natriumcarbomer kann verwendet werden. Eingesetzt wird der Gelbildner in einer Menge von bevorzugt 0,1 bis 4,0, stärker bevorzugt 0,5 bis 2,0 Gew.-% bezogen auf das Gewicht des Reservoirs.

In bevorzugter Ausführungsform besteht das Polyisobutylen des Reservoirs aus zwei unterschiedlichen Arten von Polyisobutylen mit unterschiedlichen Molekulargewichten. Dies bedeutet, dass eine Art des Polyisobutylens einen Peak bei der Verteilung des Molekulargewichts aufweist, der sich von dem Peak des Molekulargewichts der anderen Art des Polyisobutylens unterscheidet. Damit haben die beiden Polyisobutylene ein unterschiedliches mittleres Molekulargewicht (gewichtsgemitteltes mittleres Molekulargewicht M_{W}). Bevorzugt hat das eine Polyisobutylen (höhermolekulares Polyisobutylen) ein mittleres Molekulargewicht von 150.000 bis 10.000.000, besonders bevorzugt von 500.000 bis 10.000.000, und das zweite Polyisobutylen hat ein geringeres mittleres Molekulargewicht (geringermolekulares Polyisobutylen) im Bereich von 15.000 bis 100.000, vorzugsweise von 20.000 bis 80.000. Das gewichtsgemittelte mittlere Molekulargewicht M_{W} wird in der Regel über GPC bestimmt. Vor allem das Polyisobutylen mit dem niedrigeren mittleren Molekulargewicht ist verantwortlich für die Klebrigkeit des Pflasters. Die beiden Polyisobutylene mit unterschiedlichem Molekulargewicht können miteinander gemischt werden, wobei das Verhältnis von dem Polyisobutylen mit höherem Molekulargewicht zu dem Polyisobutylen mit geringerem Molekulargewicht sich bevorzugt in dem Bereich von 0,05:1 bis 20:1, besonders bevorzugt von 0,5:1 bis 2:1, insbesondere etwa 1:1 bewegt, entsprechende Gemische aus Polyisobutylenen verschiedener Molekulargewichte sind aber auch kommerziell verfügbar. Ein besonders geeignetes Handelsprodukt ist das Produkt DuroTak^{®}87-616A der Firma National Starch.

Bezogen auf das Gesamtgewicht des Reservoirs beträgt der Anteil des Polyisobutylens, bevorzugt der beiden Polyisobutylene mit unterschiedlichem Molekulargewicht, bevorzugt 30 bis 80 Gew.-%, z.B. 40 bis 65 Gew.-%, bevorzugt 50 bis 60 Gew.-%, bezogen auf das Gesamtgewicht des Reservoirs.

Sofern im Rahmen dieser Beschreibung von dem "Gesamtgewicht des Reservoirs" oder einer auf das Reservoir bezogenen Menge, etc. gesprochen wird, ist hierunter immer das Trockengewicht, also das Gewicht des Reservoirs in dem anwendungsfertigen Pflaster, zu verstehen, sofern nichts anderes offenbart oder ersichtlich ist.

Wesentlich für die erfindungsgemäßen transdermalen therapeutischen Systeme ist, dass das Reservoir so viel Wirkstoff enthält, dass dieses Wirkstoffpartikel enthält.

Bei der Herstellung des erfindungsgemäßen Pflasters wird der Wirkstoff bevorzugt in mikronisierter Form mit einer mittleren Teilchengröße von 50 µm oder weniger, bevorzugt mit einer mittleren Teilchengröße von 20 µm oder weniger, eingesetzt. Auch in der Reservoirschicht des Pflasters (der Matrixschicht) liegt der Wirkstoff in mikronisierter Form vor, allerdings können sich durch Umlagerungsreaktionen bei der Lagerung des Pflasters geringfügige Abweichungen der Teilchengröße ergeben. Auch innerhalb des Pflasters beträgt die mittlere Teilchengröße der Wirkstoffpartikel aber bevorzugt weniger als 100 µm, stärker bevorzugt weniger als 50 µm und insbesondere etwa 20 µm oder darunter. Zur Herstellung des erfindungsgemäßen Pflasters wird bevorzugt mikronisiertes Fentanyl mit einer mittleren Teilchengröße von 1 µm oder mehr, stärker bevorzugt 2 µm oder mehr, eingesetzt. Diese mittleren Teilchengrößen finden sich bevorzugt ebenfalls in den fertig hergestellten Pflastern. In der Reservoirschicht des transdermalen therapeutischen Systems kann die Teilchengröße und die Teilchengrößenverteilung der Wirkstoffteilchen durch übliche Lichtmikroskopie bestimmt werden. Die Auswertung erfolgt über übliche Computerprogramme (Bildverarbeitungssysteme), die in der Regel auf die verwendeten Mikroskope abgestimmt sind. Die Teilchengröße bezieht sich auf den Teilchendurchmesser, sofern nichts anderes angegeben oder offensichtlich ist.

Als Ausgangsmaterial für das mikronisierte Fentanyl wird handelsübliches Fentanyl eingesetzt, das an sich für die klinische Anwendung geeignet ist. Derartiges Fentanyl hat üblicherweise eine Verteilung der Partikelgröße dergestalt, dass 100% der Partikel kleiner sind als 675 µm. Etwa 90% der Partikel sind kleiner als etwa 90 µm und 50% der Partikel sind kleiner als etwa 25 µm.

Erfindungsgemäß kann jedes bekannte Mikronisierungsverfahren verwendet werden, das die gewünschte Teilchengröße zur Verfügung stellt. Bevorzugt wird Fentanyl eingesetzt, das mittels einer üblichen "jet mill" mikronisiert wurde, z.B. einer jet mill vom Typ AS der Hosokawa Alpine AG.

Durch das erfindungsgemäß eingesetzte Mikronisierungsverfahren wird die Größe der Fentanylpartikel bevorzugt so verändert, dass die mittlere Teilchengröße in den vorstehend angegebenen Bereichen liegt. Bevorzugt ist ebenfalls, dass 100% der Partikel kleiner sind als 50 µm, insbesondere als 20 µm. Etwa 90% der mikronisierten Partikel sind bevorzugt kleiner als 12 µm und etwa 50% der Partikel sind bevorzugt kleiner als 6 µm.

Für die Bestimmung der Teilchengröße bzw. der Teilchengrößenverteilung des Wirkstoffes gibt es verschiedene Verfahren, beispielsweise Lichtstreuverfahren, wie sie in den Geräten der Firma Malvern Instruments, z.B. dem "Malvern MasterSizer X", verwendet werden, mechanische Siebrüttelverfahren, wie sie die Firma FMC zur Bestimmung der Korngrößenverteilung ihrer AVICEL PH^{®}-Produkte verwendet, oder auch "air jet"-Siebanalysen, die beispielsweise mit einem ALPINA^{®}-"air jet" Modell 200 ausgeführt werden können. Bevorzugt erfolgt die Bestimmung der Partikelgrößen und -verteilungen mikroskopisch durch Lichtmikroskopie und eine geeignete Bildverarbeitungssoftware.

Sofern nicht anders angegeben, werden die (mittleren) Teilchengrößen bzw. Teilchengrößenverteilungen mit einem optischen Mikroskop, daran angebrachter Kamera und automatisierter Auswertesoftware bestimmt. Hierbei sind die dafür vorgesehenen Testbedingungen einzuhalten, die in der Regel von den Herstellern der Mikroskope vorgegeben werden.

Definiert man den Wirkstoff über die Angabe der mittleren Teilchengröße und die Teilchengrößenverteilung, weist der erfindungsgemäß eingesetzte mikronisierte Wirkstoff bevorzugt eine mittlere Teilchengröße von 20 µm oder weniger auf, und es ist bevorzugt, dass der Wirkstoff eine Korngrößenverteilung (Teilchengrößenverteilung) hat, bei der weniger als 10% der Teilchen eine Größe von 25 µm oder darüber haben und weniger als 10% der Teilchen eine Größe von 1 µm oder darunter haben. Noch weiter bevorzugt ist ein Wirkstoff mit einer mittleren Teilchengröße von 15 µm. Bevorzugt hat ein derartiger Wirkstoff eine Korngrößenverteilung, bei der weniger als 2% der Teilchen eine Größe von 20 µm oder darüber haben und weniger als 50% der Teilchen eine Größe von 5 µm oder darunter haben. Die Korngrößenverteilung für diesen Wirkstoff sollte so eng wie möglich sein.

Auf der der menschlichen Haut beim Gebrauch abgewandten Seite des Reservoirs befindet sich eine Rückschicht, die in bevorzugter Ausführungsform okklusiv, also abschließend ist. Derartige Rückschichten können in bevorzugter Ausführungsform aus Polyolefinen, insbesondere Polyethylen, oder aus Polyester sowie Polyurethanen bestehen. Auch Schichten, die mehrere verschiedene Polymere übereinander angeordnet beinhalten, sind vorteilhafterweise einsetzbar. Ein besonders bevorzugtes Material für die Rückschicht ist ein Polyolefin, das von der Firma Mylan Technologies Inc. unter der Bezeichnung Mediflex^{®}1000 vertrieben wird. Andere geeignete Materialien umfassen Cellophan, Celluloseacetat, Ethylcellulose, mit Weichmachern versehene Vinylacetatvinylchloridcopolymere, Ethylenvinylacetatcopolymere, Polyethylenterephthalat, Nylon, Polyethylen, Polypropylen, Polyvinylidenchlorid, Ethylenmethacrylatcopolymer, Papier, das gegebenenfalls beschichtet sein kann, textile Gewebe, Aluminiumfolie und Polymer-Metall-Kompositmaterialien. Besonders bevorzugt sind Polyesterfolien, wie Polyethylentherephthalatfolien. Die Dicke der Rückschicht ist, wie im Stand der Technik üblich, z.B. 10 µm bis 50 µm, z.B. etwa 20 µm nominelle Dicke.

Auf der Rückschicht des Pflasters befindet sich bevorzugt eine Abdeckschicht, die insbesondere verhindern soll, dass das Pflaster mit der Verpackung verklebt, falls geringe Mengen Polyisobutylen oder Klebstoff austreten. Die Abdeckschicht liegt bevorzugt lose auf der Rückschicht auf und wird durch elektrostatische Kräfte gehalten. Derartige Abdeckschichten sind im Stand der Technik bekannt, z.B. aus der EP 1 097 090, auf die insoweit vollinhaltlich Bezug genommen wird. Die Abdeckschicht ist zumindest auf der auf der Rückschicht aufliegenden Seite antihaftbeschichtet., z.B. silikonisiert oder fluoriert.

Die Herstellung eines bevorzugten transdermalen therapeutischen Systems erfolgt, indem man zunächst die Komponenten für das Reservoir, also Fentanyl und den Gelbildner, in einem organischen Medium wie Heptan dispergiert und das Mineralöl und Polyisobutylen in einem organischen Medium, bevorzugt dem gleichen wie vorher, miteinander mischt. Anschließend werden Fentanyl und Gelbildner in dem Gemisch aus Polyisobutylen und Mineralöl dispergiert. Bei der Herstellung des Reservoirs wird bevorzugt ein flüchtiges organisches Medium, wie beispielsweise Heptan, eingesetzt. Diese Mischung wird dann als gleichmäßige Schicht auf der Rückschicht aufgebracht und getrocknet. Falls eine Membran aufgebracht werden soll, wird diese nun auf der der Rückschicht gegenüberliegenden Seite des Reservoirs aufgebracht.

In einem separaten Arbeitsschritt wird gegebenenfalls die (wirkstofffreie) Klebstoffschicht auf die Abziehfolie aufgebracht und trocknen gelassen.

Die in den beiden Verfahrensschritten erhaltenen Komponenten werden anschließend miteinander laminiert, und zwar so, dass die Klebstoffschicht auf die Membran aufgebracht wird, falls eine derartige Membran vorgesehen ist. In denjenigen Ausführungsformen, in denen keine Membran eingesetzt wird, wird die Klebstoffschicht direkt auf das Reservoir laminiert (falls in diesem Fall eine Klebstoffschicht vorhanden ist). Anschließend können aus der fertigen laminierten Folie Stücke gewünschter Größe ausgestanzt werden und verpackt werden.

Bei den einzelnen Verfahrensschritten werden die organischen Lösungsmittel, die erforderlich sind, um das Polyisobutylen in Lösung zu bringen und die anderen Bestandteile zu dispergieren, dadurch entfernt, dass die Produkte ansteigenden Temperaturen, gegebenenfalls auch unter Anwendung eines Unterdrucks, ausgesetzt werden.

Mit dem erfindungsgemäßen transdermalen therapeutischen System können die Blutplasmaspiegel sehr gut eingestellt werden. Erfindungsgemäß bevorzugt sind daher transdermale therapeutische Systeme, wie sie in dieser Anmeldung beschrieben und beansprucht werden, die bei Verabreichung an einen Patienten im steady-state einen Blutplasmaspiegel im Bereich von 200 pg/ml bis 500 pg/ml, bevorzugt von 250 pg/ml bis 350 pg/ml (pg = Pikogramm) zur Verfügung stellen. Bevorzugt sind insbesondere auch diejenigen transdermalen therapeutischen Systeme, wie sie in dieser Anmeldung beschrieben und in den Ansprüchen beansprucht sind, bei denen Folgendes gilt:

Legt man durch die Blutplasmaspiegelwerte im Bereich von 30 Stunden bis 70 Stunden eine Regressionsgerade (mindestens ein Messpunkt alle 5 Stunden), so weicht der Blutplasmaspiegelwert auf der Regressionsgeraden bei 30 Stunden von dem Blutplasmaspiegelwert auf der Regressionsgeraden bei 70 Stunden um nicht mehr als 10% voneinander ab.

Dies zeigt die sehr hohe Konstanz der Blutplasmaspiegelwerte des Fentanyls, die mit den erfindungsgemäßen Formulierungen erhalten werden können.

Die folgenden Beispiele erläutern die Erfindung, sind aber nicht einschränkend.

### Beispiel 1

Für die Herstellung eines transdermalen Pflasters wurde zunächst Fentanyl mit einer mittleren Partikelgröße von 20 µm (Gesellschaft für Mikronisierung mbH, jet mill, AS, Hosokawa Alpine AG) in Heptan mit dem Polyisobutylen (DuroTak^{®}87-616A), Siliziumdioxid (Cab-O-Sil M-5P) als Gelbildner und Mineralöl "Klearol" als Weichmacher dispergiert. Die Mischung wurde als dünne Schicht auf die Rückschicht aufgetragen und trocknen gelassen, so dass sich ein Flächengewicht von etwa 55 g/m² ergab. Hierauf wurde als Membran ein mikroporöser Polypropylen-Film (Celgard 2400) aufgebracht.

Parallel hierzu wurde nur Polyisobutylen (DuroTak^{®}87-616A, gelöst in Heptan) in dünner Schicht auf die Abziehschicht aufgebracht und trocknen gelassen, so dass sich ein Flächengewicht von etwa 30 g/m² ergab. Die Toleranz der Flächengewichte betrug 10%.

Nachdem beide Schichten getrocknet waren, wurden die beiden Schichten miteinander laminiert, wobei die Membran mit dem Reservoir durch Anwendung geeigneten Drucks verbunden wurde.

Anschließend wurden rechteckige Pflaster mit abgerundeten Kanten in einer Größe von 10 cm² ausgestanzt und mit der Abdeckfolie verpackt. Das fertige Produkt wies die folgende Zusammensetzung auf:

| **Name des Bestandteils** | **Nominelle Freisetzung 25 µg/h Patchgröße 10 cm²** | **Konzentration (%) des anwendungsfertigen (trockenen) Reservoirs** |
|---|---|---|
| **Fentanylenthaltendes Reservoir** | | |
| Fentanyl | 4,95 mg | 9% |
| DuroTak^{®}87-616A | 30,0 mg | 54,5% |
| Cab-O-Sil M-5P | 0,55 mg | 1% |
| Klearol (Mineralöl) | 19,5 mg | 35,5% |
| Heptan* | --- | --- |

| **Klebeschicht** | | |
|---|---|---|
| DuroTak^{®}87-616A | 30,0 mg | 100% |
| Heptan* | --- | --- |

| **Schichtmaterialien** | | |
|---|---|---|
| Celgard 2400 | 10,0 cm² | |
| Hostaphan MN 19 MED | 10,0 cm² | |
| Primeliner 100 µm PET C1S | 15 cm² | |
| PETP Film, 36 µm, transparent | 15 cm² | |
| Aluminiumverpackung, weiß | 1 Beutel | |

| | | |
|---|---|---|
| * im fertigen Produkt nicht vorhanden | | |

Die in dem Beispiel eingesetzten Komponenten können näher beschrieben werden wie folgt:

| **Komponentenbezeichnung** | **chemische Beschreibung** | **Funktion** |
|---|---|---|
| DuroTak^{®}87-616A | Polyisobutylenklebemittel gelöst in n-Heptan | Haftklebstoff |
| Cab-O-Sil M-5P | kolloidales Siliziumdioxid | gelbildendes Mittel |
| Klearol | leichtes Mineralöl | Weichmacher |
| Celgard 2400 | mikroporöser Polypropylenfilm | Membran |
| Hostaphan MN 19 MED | Polyethylenterephthalatfilm mit 19 µm nomineller Dicke | Rückschicht |
| Primeliner 100 µm PET C1S | Polyesterfilm mit 100 µm Nominaldicke, eine Seite silikonisiert | Abziehschicht |
| PETP-Film, 36 µm, transparent | Polyesterfilm mit nominaler Dicke, 36 µm, eine Seite silikonisiert | Abdeckschicht |
| Aluminiumpackverpackung, weiß | Heißklebeverpackung | Verpackungsmaterial |

### Beispiel 2

Transdermale Pflaster, die gemäß der Vorgehensweise, wie in Beispiel 1 beschrieben, hergestellt wurden, wurden in einer Pilotstudie überprüft.

Bei der Pilotstudie wurden sechs gesunde freiwillige Probanden eingesetzt. Es wurden insgesamt zwei verschiedene Pflaster getestet. Als Kontrolle diente ein Produkt aus dem Stand der Technik mit der Bezeichnung Durogesic SMAT 25 µg/h, ein monolithisches transdermales System mit Polyacrylatmatrix. Dieses Pflaster wurde mit dem erfindungsgemäßen Pflaster, wie in Beispiel 1 beschrieben, verglichen. Hier beträgt die nominelle Freisetzung 25 µg Fentanyl pro Stunde.

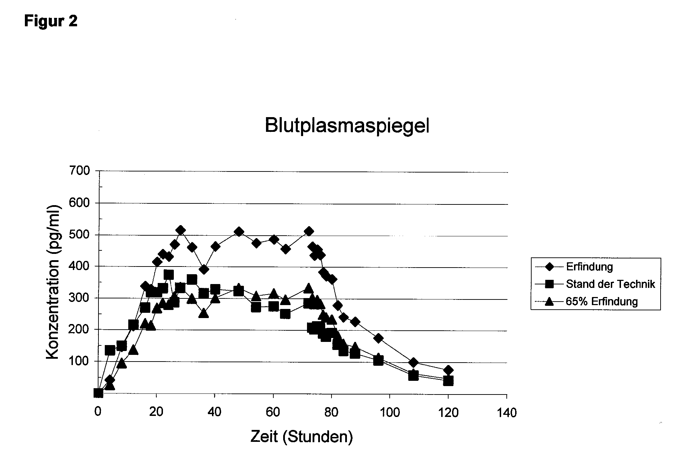

Die Pflaster wurden bei den Probanden aufgeklebt und anschließend wurden die Konzentrationen von Fentanyl im Blut gemessen. Die Mittelwerte von jeweils sechs Messungen wurden in der Freisetzungskurve gemäß Figur 2 aufgetragen.

Die Freisetzungskurve gemäß Figur 2 zeigt, dass das erfindungsgemäße Pflaster (-◆- Erfindung) eine deutlich höhere Konzentration von Fentanyl im Plasma der Probanden zeigt und dass die Konzentration über etwa drei Tage noch besser konstant gehalten wird als bei dem Polyacrylatpflaster, das als kommerzielles Produkt trotz der nicht vollständig konstanten Freisetzung eine arzneimittelrechtliche Zulassung erhalten hat.

Ein Pflaster, das nur 65% der Größe des erfindungsgemäßen Pflasters aufweist, zeigt daher einen Plasmaspiegel bei den Probanden, der dem Wert des Standes der Technik entspricht, verbunden mit der besseren Konstanz als bei dem Polyacrylatpflaster. Auch diese Werte sind in Figur 2 aufgenommen.

Dieses Beispiel zeigt deutlich, dass das erfindungsgemäße transdermale Pflaster eine erheblich verbesserte Freisetzung aufweist, verglichen mit einem Produkt des Standes der Technik. Daher können kleinere Pflaster dieselbe Wirksamkeit aufweisen wie die größeren, aus dem Stand der Technik bekannten Pflaster. Außerdem zeigt die Blutspiegelkurve, dass der Wirkstoff effizienter ausgenutzt wird.

Darüber hinaus zeigte sich überraschend, dass sich bei den erfindungsgemäßen Pflastern der bei den Polyisobutylenpflastern des Standes der Technik beschriebene Effekt, nämlich eine Zeitabhängigkeit der Freisetzung innerhalb der ersten 3 Tage nach der Verabreichung gerade nicht zeigt, sondern die Plasmakonzentration über mindestens 3 Tage konstant bleibt, ohne dass sich nach einem anfänglichen schnellen Anstieg über die ersten 20 Stunden ein langsames Abfallen der Plasmakonzentration zeigt. Dies ist überraschend und ausgesprochen vorteilhaft bei den erfindungsgemäßen Polyisobutylenpflastern, die daher sogar den derzeit am Markt vorhandenen Fentanylpflastern auf Polyacrylatbasis überlegen sind.

## Patentansprüche

1. Transdermales therapeutisches System zur Verabreichung eines Wirkstoffes durch die Haut umfassend:
(a) eine Rückschicht
(b) ein auf der Rückschicht befindliches Reservoir, umfassend den Wirkstoff, Polyisobutylen, einen Gelbildner und einen Weichmacher, wobei ein Teil des Wirkstoffs in dem Reservoir in Form ungelöster Partikel vorliegt und der Gehalt an Gelbildner in dem Reservoir höchstens 4 Gew.-% beträgt (bezogen auf das Gesamtgewicht des Reservoirs),
wobei der Wirkstoff Fentanyl oder ein Analogstoff davon ist.

2. Transdermales therapeutisches System nach Anspruch 1, **dadurch gekennzeichnet, dass** es sich bei dem Wirkstoff um Fentanyl oder Sufentanil handelt.

3. Transdermales therapeutisches System nach einem der Ansprüche 1 oder 2, bei dem auf der der Rückschicht entgegengesetzten Seite des Reservoirs eine Membran vorgesehen ist, bei der es sich um eine mikroporöse Membran handelt.

4. Transdermales therapeutisches System nach einem der Ansprüche 1 bis 3, das eine Abziehschicht (release liner) aufweist.

5. Transdermales therapeutisches System nach Anspruch 4, **dadurch gekennzeichnet, dass** das Reservoir eine Menge an Fentanyl oder Analogstoff davon enthält, die ausreichend ist, um Schmerzfreiheit zu induzieren und für einen Zeitraum von drei bis sieben Tagen aufrechtzuerhalten.

6. Transdermales therapeutisches System nach einem der Ansprüche 1 bis 5, **dadurch gekennzeichnet, dass** es sich bei dem Weichmacher um Mineralöl handelt.

7. Transdermales therapeutisches System nach einem der Ansprüche 1 bis 6, **dadurch gekennzeichnet, dass** es sich bei dem Gelbildner um kolloidales Siliziumdioxid oder um Bentonit handelt.

8. Transdermales therapeutisches System nach einem der Ansprüche 1 bis 7, **dadurch gekennzeichnet, dass** die Wirkstoffpartikel in dem Reservoir in mikronisierter Form mit einer mittleren Teilchengröße von 50 µm oder weniger, insbesondere von 20 µm oder weniger, vorliegen.

9. Transdermales therapeutisches System nach einem der Ansprüche 1 bis 8, **dadurch gekennzeichnet, dass** es sich bei dem Polyisobutylen um ein Gemisch aus zwei Polyisobutylenpolymeren mit unterschiedlichem Molekulargewicht handelt.

10. Transdermales therapeutisches System nach einem der Ansprüche 1 bis 9 **dadurch gekennzeichnet, dass** auf der Rückschicht auf der dem Reservoir entgegengesetzten Seite eine Abdeckschicht angebracht ist.

11. Transdermales therapeutisches System nach einem der Ansprüche 1 bis 10, **dadurch gekennzeichnet, dass** es sich bei der Rückschicht um eine okklusive Rückschicht handelt.

12. Transdermales therapeutisches System nach einem der Ansprüche 1 bis 11, **dadurch gekennzeichnet, dass** der Wirkstoff in dem Reservoir in einer Konzentration im Bereich von 3 Gew.-% bis 20 Gew.-% vorhanden ist, bezogen auf das Gesamtgewicht des Reservoirs.

13. Transdermales therapeutisches System nach einem der Ansprüche 1 bis 12, **dadurch gekennzeichnet, dass** der Weichmacher in einer Menge von 10 bis 60% in dem Reservoir vorhanden ist, bezogen auf das Gesamtgewicht des Reservoirs.

14. Transdermales therapeutisches System nach einem der Ansprüchen 1 bis 13, **dadurch gekennzeichnet, dass** das Polyisobutylen in einer Menge von 30 bis 80% in dem Reservoir vorhanden ist, bezogen auf das Gesamtgewicht des Reservoirs.

15. Transdermales therapeutisches System nach einem der Ansprüche 1 bis 14, **dadurch gekennzeichnet, dass** es eine Klebstoffschicht aufweist, die frei von ungelöstem Wirkstoff ist.

16. Transdermales therapeutisches System nach Anspruch 15, **dadurch gekennzeichnet, dass** es sich bei der Klebstoffschicht um ein Polyacrylat oder ein Polyisobutylen handelt.

17. Transdermales therapeutisches System nach Anspruch 15 oder 16, **dadurch gekennzeichnet, dass** die Fläche der Klebstoffschicht der Fläche des Reservoirs entspricht.
